# EUROPÄISCHE PATENTANMELDUNG

(11) **EP 2 161 024 A1**
(43) Veröffentlichungstag der Anmeldung: **10.03.2010**
(21) Anmeldenummer: 08163829.8
(22) Anmeldetag: 05.09.2008
(51) Int. Cl.: A61K 31/445, A61K 31/40, A61K 31/366, A61P 35/00

(54) **Kombinationspräparat zur Behandlung von Krebs**

(71) Anmelder: Universitätsklinikum Hamburg-Eppendorf, 20246 Hamburg (DE)
(72) Erfinder: Schumacher, Udo, 21465 Wentorf (DE); Eschenhagen, Thomas, 20257 Hamburg (DE)
(74) Vertreter: UEXKÜLL & STOLBERG

(57) **Zusammenfassung**

Die vorliegende Erfindung betrifft pharmazeutische Zusammensetzungen für die Behandlung von Krebserkrankungen, die mindestens ein Statin oder ein pharmazeutisch geeignetes Salz oder Solvat desselben sowie den Wirkstoff Astemizol oder ein pharmazeutisch geeignetes Salz oder Solvat desselben umfassen. Insbesondere betrifft die Erfindung eine pharmazeutische Zusammensetzung, die das Statin Atorvastatin und/oder das Statin Lovastatin und den Wirkstoff Astemizol bzw. pharmazeutisch geeignete Salze oder Solvate dieser Verbindungen umfassen. Die Erfindung stellt ferner Kits bereit, die diese Verbindungen umfassen.

## Beschreibung

Die vorliegende Erfindung betrifft pharmazeutische Zusammensetzungen für die Behandlung von Krebserkrankungen, die mindestens ein Statin oder ein pharmazeutisch geeignetes Salz oder Solvat desselben sowie den Wirkstoff Astemizol oder ein pharmazeutisch geeignetes Salz oder Solvat desselben umfassen. Insbesondere betrifft die Erfindung eine pharmazeutische Zusammensetzung, die das Statin Atorvastatin und/oder das Statin Lovastatin und den Wirkstoff Astemizol bzw. pharmazeutisch geeignete Salze oder Solvate dieser Verbindungen umfassen. Die Erfindung stellt ferner Kits bereit, die diese Verbindungen umfassen.

### HINTERGRUND DER ERFINDUNG

Neoplastische Erkrankungen zeichnen sich durch ein unkontrolliertes Wachstum anormaler Zellen aus, wobei sich diese Zellen unkontrolliert vermehren und Metastasen in anderen Organen bilden können. In den Industriestaaten lassen sich etwa 20% aller Todesfälle auf Krebserkrankungen zurückführen.

Die gegenwärtig in der klinischen Therapie eingesetzten Konzepte stützen sich im wesentlichen auf die chirurgische Entfernung des neoplastischen Gewebes, die Strahlentherapie und die Behandlung mit Chemotherapeutika. Eine Chemotherapie wird in der Regel eingesetzt, wenn die Erkrankung verhältnismäßig weit fortgeschritten ist. Ferner kann die Behandlung mit Chemotherapeutika im Falle von primär generalisierten Erkrankungen, wie z.B. den hämatologischen Krebserkrankungen indiziert sein. Die Behandlung mit Chemotherapeutika beruht auf der gegenüber somatischen Zellen gesteigerten Proliferationsrate von Krebszellen, die mit einer erhöhten Empfindlichkeit gegenüber cytotoxischen Substanzen einher geht. Chemotherapeutika, die sich für die Behandlung bestimmter neoplastischer Erkrankungen eignen, sind im Stand der Technik in großer Zahl beschrieben worden. Einen Überblick bietet die folgende Publikation (De Vita, Hellman & Rosenberg, Cancer: Principles and Practice of Oncology, 7. Auflage 2004, Lipincott, Williams & Wilkins).

Allerdings sprechen etwa die Hälfte aller neoplastischen Gewebe nicht oder nur unzureichend auf eine Behandlung mit den gegenwärtig verfügbaren Cytostatika an. Dies gilt insbesondere für Tumore, die sich von Lunge, Kolon, Pankreas, Leber und Niere ableiten. Diese Tumore sind im allgemeinen gegen die heute zur Verfügung stehenden Chemotherapeutika unempfindlich, da sie über Mechanismen verfügen, die eine Resistenz gegen diese Mittel bewirken. Aus diesem Grunde ist es wichtig, weitere Mittel bereitzustellen, die für die Behandlung solcher resistenten Tumore eingesetzt werden können. Ferner ist bereits frühzeitig in der klinischen Praxis bemerkt worden, dass viele neoplastische Gewebe, die zunächst empfindlich gegenüber den eingesetzten chemotherapeutischen Mitteln sind, im weiteren Behandlungsverlauf diese Empfindlichkeit verlieren und zunehmend resistent werden. Dies hat zur Folge, dass sich die behandelten Tumoren nach mehreren Chemotherapiezyklen nicht mehr hinsichtlich ihres Wachstums beeinflussen lassen.

Vor diesem Hintergrund liegt der vorliegenden Erfindung somit die Aufgabe zugrunde, neue pharmazeutische Zusammensetzungen und Präparate bereitzustellen, mit deren Hilfe eine effektive Behandlung verschiedenartiger Krebserkrankungen möglich ist. Erfindungsgemäß wird diese Aufgabe durch die pharmazeutischen Zusammensetzungen und Präparate gemäß den anliegenden Ansprüchen gelöst.

Im Rahmen der vorliegenden Erfindung wurde nunmehr überraschend herausgefunden, dass Wirkstoffkombinationen aus mindestens einem Statin und Astemizol eine starke antiproliferative Wirkung auf neoplastische Zellen ausüben. Die vorliegende Erfindung stellt demgemäß in einem ersten Aspekt eine pharmazeutische Zusammensetzung für die Behandlung einer Krebserkrankung bereit, die die folgenden Komponenten umfaßt:
a) mindestens ein Statin oder ein pharmazeutisch geeignetes Salz oder Solvat desselben, und
b) Astemizol oder ein pharmazeutisch geeignetes Salz oder Solvat desselben.

Es ist allerdings für die Ausführung der vorliegenden Erfindung nicht zwingend notwendig, daß die oben aufgeführten Komponenten in einer einheitlichen pharmazeutischen Zusammensetzung vorliegen. Vielmehr können das Statin und Astemizol auch in getrennten Formulierungen vorliegen, die gleichzeitig oder zeitlich versetzt an den zu behandelnden Patienten verabreicht werden. Somit betrifft die Erfindung in einem zweiten Aspekt ein pharmazeutisches Kombinationspräparat, umfassend:
a) mindestens ein Statin oder ein pharmazeutisch geeignetes Salz oder Solvat desselben, und
b) Astemizol oder ein pharmazeutisch geeignetes Salz oder Solvat desselben,
   für die gleichzeitige oder zeitlich getrennte Anwendung in der Therapie.

Dies bedeutet, daß das Statin gemeinsam mit dem Astemizol als einheitliche Formulierung, d.h. als einheitliche pharmazeutische Zusammensetzung, verabreicht werden kann. Die beiden Wirkstoffe der erfindungsgemäßen Kombination können beispielsweise in vitro, d.h. vor der Verabreichung an den Patienten, miteinander zu einer einheitlichen wie oben beschriebenen Darreichungsform kombiniert werden, solange keiner der beiden Bestandteile durch das Vermischen mit dem jeweils anderen Bestandteil der Kombination einen Verlust bezüglich der antiproliferativen Aktivität erleidet. So lassen sich beispielsweise das Statin (oder die Statine) und Astemizol, die in einer lyophilisierten Mischung vorliegen, durch Zugabe eines geeigneten Lösungsmittel zu einer Infusionslösung oder Injektionslösung rekonstituieren, die beide Wirkstoffe umfaßt. Darüber hinaus kann die pharmazeutische Zusammensetzung direkt als einheitliche Dosierungsform bereitgestellt werden, z.B. in Form einer Tablette für die orale Verabreichung. Gemäß einer Ausführungsform der vorliegenden Erfindung handelt es sich bei der pharmazeutischen Zusammensetzung um eine einheitliche Zusammensetzung, bei der das Statin (oder die Statine) und Astemizol oder deren Salze in einer einheitlichen Darreichungsform verabreicht werden.

Alternativ dazu können das Statin (oder die Statine) und Astemizol in getrennten Formulierungen vorliegen, die zum selben Zeitpunkt oder zu unterschiedlichen Zeitpunkten an den Patienten verabreicht werden. So können beispielsweise das Statin (oder die Statine) und Astemizol an verschiedenen Tagen eines Behandlungszyklus verabreicht werden. Das Statin kann beispielsweise im Rahmen eines sich wiederholenden einwöchigen Behandlungszyklus täglich verabreicht werden, wobei Astemizol lediglich an einem bestimmten Tag dieses Zyklus verabreicht wird. Sofern das Statin oder die Statine und Astemizol zeitlich getrennt voneinander verabreicht werden sollen, ist es zweckmäßig, die Wirkstoffe in getrennten Verpackungseinheiten (z.B. in mehreren Ampullen) bereitzustellen. Dabei können das Statin (oder die Statine) und Astemizol in gleichartigen oder unterschiedlichen Darreichungsformen vorliegen, die nachstehend genauer beschrieben werden.

Aufgrund der antiproliferativen Wirkung der Kombination aus Statin und Astemizol lassen sich die pharmazeutischen Zusammensetzungen und Präparate der vorliegenden Erfindung in zweckmäßiger Weise zur Behandlung von verschiedenen Krebserkrankungen einsetzen. Dabei sind die Zusammensetzungen und Präparate hinsichtlich ihrer Anwendung nicht auf bestimmte Krebsarten beschränkt. Krebserkrankungen, die wirksam durch die erfindungsgemäßen Zusammensetzungen und Präparate behandelt werden können, umfassen Bronchialkarzinome (kleinzelliges Bronchialkarzinom und großzelliges Bronchialkarzinom), Kolonkrebs, Brustkrebs, Prostatakrebs, Leberkrebs, Pankreaskrebs, Blasenkrebs, Hautkrebs (z.B. Melanome), Eierstockkrebs, hämatologische Krebserkrankungen, Krebserkrankungen des Gehirns, Magenkrebs, Nierenkrebs, Uteruskrebs, Knochenkrebs, Ösophaguskrebs, Kaposi-Sarkom, oropharyngale Krebserkrankungen, Hodenkrebs, Schilddrüsenkrebs, Lymphome, adenokortikale Krebserkrankungen, Gallenblasenkrebs, multiples Myelom, Dünndarmkrebs, Analkrebs, Bauchspeicheldrüsenkrebs, Burkitt-Lymphom, Gallengangkrebs, Gebärmutterhalskrebs, Gebärmutterkörperkrebs, Harnröhrenkrebs, Kehlkopfkrebs, Knochenkrebs, Morbus Hodgkin, Non-Hodgkin-Lymphom, Wilms-Tumor, Plasmocytom oder Retionblastom.

Gemäß einer bevorzugten Ausführungsform der vorliegenden Erfindung ist die zu behandelnde Krebserkrankung ein Bronchialkarzinom. Dabei handelt es sich um eine Neoplasie, die von den Schleimhautzellen ausgeht, welche das Innere der Bronchien oder Bronchiolen auskleiden. Das Bronchialkarzinom macht über 95% aller bösartigen Tumoren der Lunge aus. Symptome des Bronchialkarzinoms sind häufig anhaltender Husten, Atemnot und Schmerzen im Brustkorb. Zu den Bronchialkarzinomen zählen das Plattenepithelkarzinom, das Adenokarzinom, das großzellige Bronchialkarzinom sowie das kleinzellige Bronchialkarzinom. Die Bronchialkarzinome unterscheiden sich hinsichtlich ihrer histologischen Eigenschaften insbesondere durch ihr Wachstums- und Ausbreitungsverhalten.

Gemäß einer besonders bevorzugten Ausführungsform der vorliegenden Erfindung handelt es sich bei der zu behandelnden Krebserkrankung um das kleinzellige Bronchialkarzinom. Diese Art von Bronchialkarzinom macht ungefähr 20-25% aller diagnostizierten Bronchialkarzinome aus. Das kleinzellige Bronchialkarzinom geht von den neuroendokrinen APUD-Zellen aus und siedelt sich meist zentral in der Lunge an. Kleinzellige Bronchialkarzinome stellen eine besonders aggressive Krebsart dar, da ihre frühe lymphogene und hämatogene Metastasierung dazu führt, daß sich in der Regel schon vor der Entdeckung des eigentlichen Karzinoms Metastasen gebildet haben. Das kleinzellige Lungenkarzinom wächst sehr schnell und breitet sich rasch über Blut- und Lymphgefäße in Lunge, Skelett, Knochenmark, Leber und Gehirn aus. Das kleinzellige Bronchialkarzinom ist praktisch inoperabel. Auch die derzeit verfügbaren Therapieansätze durch Bestrahlung und/oder Chemotherapie führen nicht zu wesentlichen Erfolgen. In der Regel führt das kleinzellige Bronchialkarzinom bei praktisch allen Patienten innerhalb von zwei Jahren nach Stellung der Diagnose zum Tod. Die nichtkleinzelligen Bronchialkarzinome, zum Beispiel das Plattenepithelkarzinom, das Adenokarzinom und das großzellige Bronchialkarzinom, wachsen und metastasieren im Vergleich zum kleinzelligen Lungenkarzinom wesentlich langsamer und sind daher mit höheren Therapieerfolgen assoziiert.

Erfindungsgemäß umfassen die bereitgestellten pharmazeutischen Zusammensetzungen und Präparate mindestens ein Statin. Wie vorliegend verwendet, bezeichnet der Begriff Statin einen pharmazeutischen Wirkstoff, der zu einer (vollständigen oder teilweisen) Hemmung des Enzyms HMG-CoA-Reduktase führt. Da das Enzym HMG-CoA-Reduktase zur Synthese von Cholesterin benötigt wird, werden Statine in der klinischen Praxis als Cholesterinsenker bei Fettstoffwechselstörungen eingesetzt. Erfindungsgemäß können verschiedene kommerziell erhältliche Statine zur Herstellung der pharmazeutischen Zusammensetzungen und Präparate verwendet werden. Solche Statine umfassen insbesondere Atorvastatin, das unter dem Handelsnamen Sortis®, Torvast® oder Lipitor® vertrieben wird (siehe US Patent 5,273,995). Weitere geeignete Statine umfassen Cerivastatin (US Patent 5,502,199), das unter dem Handelsnamen Lipobay®, Baycol® oder Zenas® erhältlich ist; Pravastatin (US Patent 4,346,227), das unter dem Handelsnamen Mevalotin®, Pravasin®, Pravachol®, Selektine® oder Lipostat® vertrieben wird; Lovastatin (US Patent 4,231,938), das unter dem Handelsnamen Mevinacor®, Altocor®, Altoprev® oder Statosan® erhältlich ist; Fluvastatin (US Patent 4,739,073), das unter dem Handelsnamen Locol®, Cranoct®, Lescol® oder Fractal® vertrieben wird; Rosuvastatin (US Patent 5,260,440), das unter dem Handelsnamen Crestor® erhältlich ist; und Simvastatin (US Patent 4,444,784), das unter dem Handelsnamen Zocor®, Sivastin®, Lipovas®, Lodales®, Lipex® oder Simvotin® vertrieben wird. Die Statine können in kristalliner Form, in flüßig-kristalliner Form, in nicht-kristalliner oder in amorpher Form vorliegen. Der Fachmann wird erkennen, daß auch Derivate der genannten Statine verwendet werden können, solange die gewünschte pharmakologische Aktivität, d.h. die antiproliferative Wirkung bei Gabe in Kombination mit Astemizol, erhalten bleibt.

Gemäß einer bevorzugen Ausführungsform ist das Statin, das im Rahmen der pharmazeutischen Zusammensetzung und Präparate verwendet wird, ein Statin, von dem bekannt ist, dass es bereits bei alleiniger Gabe eine cytostatische und/oder cytotoxische Wirkung auf Krebszellen ausübt. Eine solche Wirkung wurde für die Statine Atorvastatin und Lovastatin beschrieben (Kamat und Nelkin, Urology (2005), 66(6), 1209-12; Rao et al. (1998), Oncogene 17(18), 2393-402). Gemäß einer besonders bevorzugen Ausführungsform handelt es sich bei dem in der pharmazeutischen Zusammensetzung/Präparat zu verwendenden Statin um Atorvastatin oder um ein pharmazeutisch geeignetes Salz oder Solvat von Atorvastatin. Atorvastatin ist derzeit das weltweit am häufigsten verkaufte Statin zur Senkung des Cholesterinspiegels. Es ist in Kombination mit dem Wirkstoff Amlodipin zur Behandlung von Angina pectoris, Arteriosklerose, kombiniertem Bluthochdruck und Hyperlipidämie vorgeschlagen worden (siehe EP 1 491 193). Auch die Behandlung von Diabetes mittels Atorvastatin ist im Stand der Technik beschrieben worden (siehe EP 1 714 648). Die internationale Patenanmeldung WO 2006/017185 offenbart eine Wirkung von Atorvastatin bei der Behandlung verschiedener Krebserkrankungen. Die IUPAC Bezeichnung von Atorvastatin lautet (3R,5R)-7-[2-(4-Fluorphenyl)-5-isopropyl-3-phenyl-4-(phenylcarbamoyl)pyrrol-1-yl]-3,5-dihydroxyheptansäure. Atorvastatin weist die folgende Strukturformel auf:

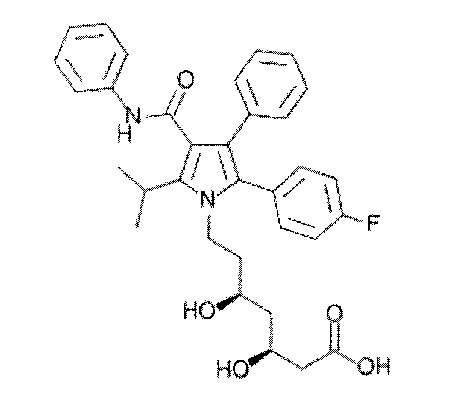

Für den Fachmann wird ersichtlich sein, dass die oben dargestellte Struktur an einer oder an mehreren Stellen substituiert oder anderweitig modifiziert werden kann, solange diese Veränderungen die antiproliferative Wirkung von Atorvastatin, die in Kombination mit Astemizol beobachtet werden kann, nicht wesentlich beeinträchtigen und auch unter toxischen Gesichtspunkten nicht zu nachteiligen Ergebnissen führen. Beispielsweise können eine oder mehrere Wasserstoffatome der C-H Bindungen des heterocyclischen Ringsystems durch Halogenatome, wie beispielsweise Chlor-, Brom- oder Iod-Atome, substituiert sein. Ferner kann der Wasserstoff der C-H Bindungen auch gegen eine Alkylgruppe, wie beispielsweise Methyl, Ethyl oder Propyl ersetzt werden.

Gemäß einer weiteren besonders bevorzugten Ausführungsform handelt es sich bei dem in der pharmazeutischen Zusammensetzung/Präparat zu verwendenden Statin um Lovastatin oder um ein pharmazeutisch geeignetes Salz oder Solvat von Lovastatin. Die IUPAC Bezeichnung von Lovastatin lautet [8-[2-(4-hydroxy-6-oxo-oxan-2-yl)ethyl]-3,7-dimethyl-1,2,3,7,8,8a-hexahydronaphthalen-1-yl]-2-methylbutanoat. Lovastatin weist die folgende Strukturformel auf:

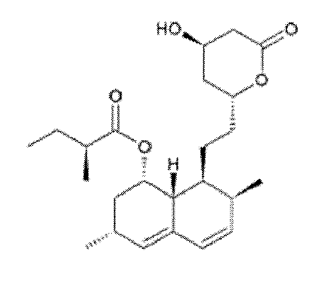

Auch die Struktur von Lovastatin kann an einer oder an mehreren Stellen substituiert oder modifiziert sein, solange die antiproliferative Wirkung der Verbindung, die in Kombination mit Astemizol beobachtet werden kann, nicht wesentlich beeinträchtigt wird und die resultierende modifizierte Verbindung sich unter toxischen Gesichtspunkten weiterhin für die Verwendung als Medikament eignet. Ein oder mehrere Wasserstoffatome der C-H Bindungen des heterocyclischen Ringsystems in der obigen Strukturformel können gegen ein Halogenatom (Cl, Br, I) substituiert sein. Ferner kann der Wasserstoff dieser C-H Bindungen auch durch eine Alkylgruppe, wie beispielsweise Methyl, Ethyl oder Propyl ersetzt sein.

Die erfindungsgemäß bereitgestellten pharmazeutischen Zusammensetzungen und Präparate umfassen neben einem oder mehreren Statinen den Wirkstoff Astemizol oder ein pharmazeutisch geeignetes Salz oder Solvat desselben. Die IUPAC Bezeichnung für Astemizol lautet 1-[(4-Fluorphenyl)methyl]-N-[1-[2-(4-methoxyphenyl)ethyl]-4-piperidyl]benzoimidazol-2-amin. Astemizol weist die nachfolgende Strukturformel auf:

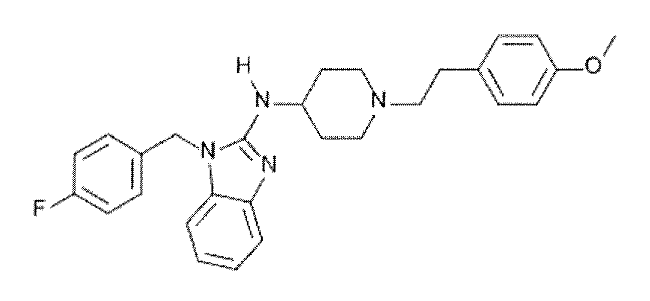

Erfindungsgemäß kann das Astemizol an einer oder an mehreren Stellen substituiert oder anderweitig modifiziert sein, solange diese Veränderungen die antiproliferative Wirkung der Substanz, die in Kombination mit einem Statin wie Atorvastatin beobachtet wird, nicht wesentlich beeinträchtigen und auch keine für den Patienten nicht tolerierbare toxische Nebenwirkungen nach sich zieht. Beispielsweise können eine oder mehrere Wasserstoffatome der C-H Bindungen des heterocyclischen Ringsystems mit einem Halogenatom, wie beispielsweise einem Chlor-, Brom- oder Iod-Atom, substituiert sein. Ferner kann der Wasserstoff dieser C-H Bindungen oder der N-H-Bindungen auch gegen eine Alkylgruppe, wie beispielsweise Methyl, Ethyl oder Propyl substituiert sein.

In einer besonders bevorzugten Ausführungsform umfaßt die erfindungsgemäße Zusammensetzung oder das erfindungsgemäße Präparat das Statin Atorvastatin oder ein pharmazeutisch geeignetes Salz oder Solvat desselben sowie Astemizol oder ein pharmazeutisch geeignetes Salz oder Solvat desselben. In einer alternativen Ausführungsform ist die erste Komponente der pharmazeutischen Zusammensetzung oder des erfindungsgemäßen pharmazeutischen Präparats das Statin Lovastatin oder ein pharmazeutisch geeignetes Salz oder Solvat desselben, und die zweite Komponente ist Astemizol oder ein pharmazeutisch geeignetes Salz oder Solvat desselben.

Der Ausdruck "pharmazeutisch geeignetes Salz" bezeichnet nicht-toxische, Säure-Additionssalze sowie Alkalimetall- bzw. Erdalkalimetallsalze. Beispielhafte Säure-Additionssalze umfassen Hydrochlorid, Hydrobromid, Sulfat, Bisulfat, Acetat, Oxalat, Phosphat, Citrat, Maleat, Fumarat, Succinat, Tartrat und Laurylsulfat. Beispielhafte Alkalimetall- bzw. Erdalkalimetallsalze umfassen Natrium-, Kalium-, Calcium- und Magnesium-Salze. Darüber hinaus können erfindungsgemäß auch Ammoniumsalze und Salze mit organischen Aminen verwendet werden. Neben dem Kalziumsalz des Atorvastatins kann auch jedes andere pharmazeutisch geeignete kationische Salz des Atorvastatins eingesetzt werden. Diese Salze sind beispielsweise solche, die durch Reaktion der freien Säureform des Atorvastatins mit einer geeigneten Base, wie beispielsweise Natriumhydroxid, Natriummethoxid, Natriumethoxid, Natriumhydrid, Kaliummethoxid, Magnesiumhydroxid, Calciumhydroxid, Cholin, Diethanolamin und weiteren, die im Stand der Technik bekannt sind, erhalten werden.

Solvate der oben genannten Verbindungen sind ebenfalls Teil der vorliegenden Erfindung. Solvate entstehen durch die Anlagerung von einem oder mehreren Lösungsmittelmolekülen an eine erfindungsgemäß vorgeschlagenen Wirkstoffverbindung (d.h. an ein Statin oder an Astemizol). Ist es das Lösungsmittel Wasser, so handelt es sich um eine Hydratation. Solvate der vorgeschlagenen Wirkstoffverbindung können durch Ionenbindungen und/oder kovalente Bindungen zusammengehalten werden.

Die erfindungsgemäßen pharmazeutischen Zusammensetzungen und Präparate können mit anderen chemotherapeutischen oder chemopräventiven Mitteln kombiniert oder in Verbindung mit diesen verwendet werden. Die Zusammensetzungen und Präparate können beispielsweise gemeinsam mit herkömmlichen, im Stand der Technik bekannten Chemotherapeutika verabreicht werden, beispielsweise zwischen zwei aufeinanderfolgenden Zyklen einer Chemotherapie. Geeignete chemotherapeutische Mittel, die im Rahmen der vorliegenden Erfindung gemeinsam den Zusammensetzungen und Präparaten angewendet werden können, umfassen die gegenwärtig im Stand der Technik verfügbaren Mittel, einschließlich alkylierende Mittel; Alkylsulfonate; Aziridine, wie z.B. Thiotepa; Ethylenimine; Anti-Metaboliten; Folsäure-Analoga, wie z.B. Methotrexat (Farmitrexat®, Lantarel®, METEX®, MTX Hexal®); Purin-Analoga, wie z.B. Azathioprin (Azaiprin®, AZAMEDAC®, Imurek®, Zytrim®), Cladribin (Leustatin®), Fludarabinphosphat (Fludara®), Mercaptopurin (MERCAP®, Puri-Nethol®), Pentostatin (Nipent®), Thioguanin (Thioguanin-Wellcome®) oder Fludarabin; Pyrimidin-Analoga, wie z.B. Cytarabin (Alexan®, ARA-cell®, Udicil®), Fluorouracil, 5-FU (Efudix®, Fluoroblastin®, Ribofluor®), Gemcitabin (Gemzar®), Doxifluridin, Azacitidin, Carmofur, 6-Azauridin, Floxuridin; Stickstoff-Lost-Derivate, wie z.B. Chlorambucil (Leukeran®), Melphalan (Alkeran®), Chlornaphazin, Estramustin, Mechlorethamin; Oxazaphosphorine, wie z.B. Cyclophosphamid (CYCLO-cell®, Cyclostin®, Endoxan®), Ifosfamid (Holoxan®, IFO-cell®) oder Trofosfamid (Ixoten®); Nitrosoharnstoffe, wie z.B. Bendamustin (Ribomustin ®), Carmustin (Carmubris®), Fotemustin (Muphoran®), Lomustin (Cecenu®, Lomeblastin®), Carmustin, Chlorozotocin, Ranimustin oder Nimustin (ACNU®); Hydroxyharnstoffe (Litalir®); Vincaalkaloide und Taxene, wie z.B. Vinblastin (Velbe®), Vindesin (Eldisine®), Vinorelbin (Navelbine®), Docetaxel (Taxotere®), oder Paclitaxel (Taxol®); Platin-Verbindungen, wie z.B. Cisplatin (Platiblastin®, Platinex®) oder Carboplatin (Carboplat®, Ribocarbo®); Sulfonsäureester, wie z.B. Busulfan (Myleran®), Piposulfan oder Treosulfan (Ovastat®); Anthrazykline, wie z.B. Doxorubicin (Adriblastin®, DOXO-cell®), Daunorubicin (Daunoblastin®), Epirubicin (Farmorubicin®), Idarubicin (Zavedos®), Amsacrin (Amsidyl®) oder Mitoxantron (Novantron®); sowie Derivate, Tautomere und pharmazeutisch aktive Salze der oben genannten Verbindungen. Auch eine Kombination der erfindungsgemäßen pharmazeutischen Zusammensetzungen oder Präparate im Rahmen von antiangiogenen Behandlungsansätzen, z.B. mit dem monoklonalen Antikörper Bevacizumab (Avastin®) oder mit Tyrosinkinase-Inhibitoren, wie z.B. Sorafenib (Nexavar®) oder Sunitinib (Sutent®), ist ohne weiteres möglich. Kombinationen der Zusammensetzungen oder Präparate mit therapeutischen Antikörpern, wie z.B. Trastuzumab (Herceptin®), Gemtuzumab (Mylotarg®), Panitumumab (Vectibix®) oder Edrecolomab (Panorex®), sind ebenfalls erfindungsgemäß umfasst.

Die erfindungsgemäßen pharmazeutischen Zusammensetzungen oder Präparate können darüber hinaus mit einer herkömmlichen Bestrahlungstherapie kombiniert werden. Die Bestrahlung kann dabei vor oder nach Verabreichung des erfindungsgemäßen Kombinationspräparats nach im Stand der Technik bekannten Verfahren durchgeführt werden. Die Bestrahlung kann Gammastrahlen, Röntgenstrahlen sowie die von Radioisotopen ausgesandte Strahlung umfassen. Die Bestrahlungstherapie kann des weiteren Teilchenstrahlung (Elektronen, Protonen, Neutronen) umfassen. Entsprechende Dosismengen, die bei der Behandlung vom Tumoren eingesetzt werden, sind dem auf dem Gebiet der Strahlentherapie tätigen Fachmann ohne weiteres bekannt. Je nach Art des Tumors können Gesamtdosen von 20 bis 80 Gray zum Einsatz kommen.

Die erfindungsgemäßen Zusammensetzungen oder Präparate können in jeder im Stand der Technik bekannten, geeigneten Darreichungsform verabreicht werden. Solche Formulierungen richten sich im wesentlichen nach den Eigenschaften des jeweils verwendeten Statins und umfassen beispielsweise Formulierungen für eine orale, rektale, nasale oder parenterale (einschließlich subkutaner, intramuskulärer, intravenöser und intradermaler) Verabreichung. Gewöhnlich umfassen die erfindungsgemäßen pharmazeutischen Zusammensetzungen oder Präparate neben dem Statin oder den Statinen und Astemizol einen oder mehrere pharmazeutisch akzeptable Träger, die mit den übrigen Inhaltsstoffen der Zusammensetzungen physiologisch kompatibel sind. Die erfindungsgemäßen Zusammensetzungen oder Präparate können neben den eigentlichen Wirkstoffen auch weitere Hilfsstoffe, Bindemittel, Verdünnungsmittel oder ähnliche Substanzen umfassen.

Die erfindungsgemäßen Zusammensetzungen und Präparate können mittels im Stand der Technik hinreichend bekannter Verfahren hergestellt werden. Derartige Verfahren sowie geeignete Hilfsstoffe und Träger werden beispielsweise in "Remington: The Science and Practice of Pharmacy", Lippincott Williams & Wilkins; 21. Auflage (2005) beschrieben. Die erfindungsgemäßen Zusammensetzungen oder die Komponenten der erfindungsgemäßen Kombinationspräparate können beispielsweise in Form von Granulaten, Pulvern, Tabletten, Kapseln, Sirup, Suppositorien, Injektionen, Emulsionen, Suspensionen oder Lösungen vorliegen. Die Zusammensetzungen und die Komponenten der Präparate können für verschiedene Verabreichungsarten formuliert sein, beispielsweise für die orale, bukkale, sublinguale, transmukosale, rektale, subkutane intrathekale, intravenöse, intramuskuläre, intraperitoneale, nasale, intraokulare oder intraventrikuläre Verabreichung. Die erfindungsgemäßen Zusammensetzungen und die Komponenten der Präparate können darüber hinaus auch als Retardmittel formuliert werden.

Für die orale, bukkale und sublinguale Verabreichung werden in der Regel feste Formulierungen wie, z.B. Pulver, Suspensionen, Granulate, Tabletten, Pillen, Kapseln und Gelcaps verwendet. Diese können beispielsweise hergestellt werden, indem man die Wirkstoffe oder deren Salze mit mindestens einem Additiv oder mit mindestens einem Hilfsstoff vermischt. Derartige Hilfsstoffe und Träger werden beispielsweise in "Remington: The Science and Practice of Pharmacy", Lippincott Williams & Wilkins; 21. Auflage (2005) beschrieben. Als Additive oder Hilfsstoffe können beispielsweise mikrokristalline Cellulose, Methylcellulose, Hydroxypropylmethylcellulose, Casein, Albumin, Mannit, Dextran, Saccharose, Lactose, Sorbitol, Stärke, Agar, Alginate, Pectine, Kollagen, Glyceride oder Gelatine verwendet werden. Ferner können orale Dosisformen Antioxidantien (z.B. Ascorbinsäure, Tocopherol oder Cystein), Gleitmittel (z.B. Magnesiumstearat), Konservierungsmittel (z.B. Paraben oder Sorbinsäure), Sprengmittel, Bindemittel, Verdicker, Geschmacksverbesserer, Farbstoffe und ähnliche Substanzen umfassen.

Flüssige Formulierungen, die sich für die orale Verabreichung eignen, können beispielsweise als Emulsion, Sirup, Suspension oder Lösungen vorliegen. Diese Formulierungen können unter Verwendung einer sterilen Flüssigkeit als pharmazeutischer Träger (z.B. Öl, Wasser, Alkohol oder Kombinationen derselben) in Form von flüssigen Suspensionen oder Lösungen hergestellt werden. Für die orale oder parenterale Verabreichung können pharmazeutisch geeignete Tenside, Suspensionsmittel, Öle oder Emulgatoren zugefügt werden. Für den Gebrauch in flüssigen Dosisformen geeignete Öle umfassen beispielsweise Olivenöl, Sesamöl, Erdnußöl, Rapsöl und Maisöl. Geeignete Alkohole umfassen Ethanol, Isopropylalkohol, Hexadecylalkohol, Glycerin und Propylenglykol. Suspensionen können ferner Fettsäureester, wie Ethyloleat, Isopropylmyristat, Fettsäureglyceride und acetylierte Fettsäureglyceride umfassen. Ferner werden Suspensionen häufig auch mit Substanzen wie Mineralöl oder Petrolatum versetzt.

Für die Injektion geeignete Formulierungen umfassen im allgemeinen wäßrige Suspensionen oder Ölsuspensionen, die unter Verwendung eines geeigneten Dispersionsmittels und eines Suspensionsmittels hergestellt werden können. Injizierbare Formen können in Lösungsphase oder in Form einer Suspension vorliegen, die mit einem Lösungsmittel oder Verdünnungsmittel hergestellt wird. Als pharmazeutischer Träger kommen u.a. sterilisiertes Wasser, Ringer-Lösung oder eine isotonische wäßrige Kochsalzlösung in Betracht. Alternativ dazu kann man als Träger sterile Öle einsetzen, die vorzugsweise nicht flüchtig sind. Für die Injektion geeignete Dosisformen umfassen ferner Pulver, die für die Rekonstitution mit einer geeigneten Lösung geeignet ist. Beispiele hierfür sind u.a. gefriergetrocknete oder sprühgetrocknete Pulver. Für die Injektion können diese Formulierungen gegebenenfalls mit Stabilisierungsmitteln oder Tensiden versetzt werden. Die Formulierungen können durch Injektion, wie durch Bolus-Injektion oder durch kontinuierliche Infusion verabreicht werden.

Für die rektale Verabreichung können die pharmazeutischen Zusammensetzungen oder die Komponenten der Präparate beispielsweise in Form eines Suppositoriums oder einer Salbe vorliegen. Suppositorien können durch Mischen der entsprechenden Wirkstoffe mit geeigneten Trägern hergestellt werden. Geeignete pharmazeutische Träger umfassen beispielsweise Kakaobutter, Glyceride oder Polyethylenglykol. Diese Substanzen liegen bei normalen Lagerungstemperaturen in fester Form vor, wohingegen sie bei der Freisetzung im Rektum in die flüssige Form übergehen. Bei der Herstellung von Suppositorien können auch Öle eingesetzt werden.

Die am besten für die jeweilige Therapie geeigneten Verabreichungsarten und die zu verabreichenden Mengen von Statin und Astemizol können vom Fachmann problemlos mittels Routineverfahren ausfindig gemacht werden. Faktoren, die die jeweilige Menge von Statin und Astemizol in der zu verabreichenden pharmazeutischen Zusammensetzung beeinflußen, umfassen die Art und Schwere der Krebserkrankung, das Alter, Körpergewicht, Geschlecht und den allgemeinen Gesundheitszustand des zu behandelnden Patienten, die gleichzeitige Verabreichung anderer therapeutischer Mittel und andere Faktoren.

Geeignete pharmazeutische Zusammensetzungen oder Präparate zur humanen Verabreichung werden in der Regel 0,01 mg bis 10 mg Statin pro kg Körpergewicht des Patienten umfassen. Vorzugsweise liegt die Menge des Statins im Bereich von 0,1 mg bis 5 mg pro kg Körpergewicht des Patienten und noch stärker bevorzugt im Bereich von 0,5 mg bis 1 mg pro kg Körpergewicht des Patienten. Die Menge an Astemizol in den pharmazeutischen Zusammensetzungen oder Präparaten wird üblicherweise von 0,01 mg bis 50 mg pro kg Körpergewicht des Patienten betragen. Vorzugsweise wird die Menge an Astemizol in den erfindungsgemäßen Zusammensetzungen oder Präparaten im Bereich von 0,05 mg bis 25 mg pro kg Körpergewicht des Patienten und noch stärker bevorzugt im Bereich von 0,1 mg bis 10 mg pro kg Körpergewicht des Patienten betragen.

Die therapeutische Wirksamkeit der erfindungsgemäßen pharmazeutischen Zusammensetzungen und Präparate kann anhand von im Stand der Technik bekannten Parametern bewertet werden. Diese Parameter umfassen u.a. die Wirksamkeit der erfindungsgemäßen Zusammensetzung bei der Beseitigung von Tumoren, die Ansprechrate, die Zeit bis zum Fortschreiten der Erkrankung und die Überlebensrate der behandelten Patienten. Eine gegen einen Tumor gerichtete Wirkung kann sich durch eine Inhibierung des Tumorwachstums, eine Verzögerung des Tumorwachstums, eine Verkleinerung des Tumors, eine Verringerung der Anzahl der Tumorzellen, eine Verlängerung der Zeitspanne bis zum Einsetzen eines erneuten Tumorwachstums sowie eine Verzögerung im Fortschreiten der Erkrankung darstellen.

Vorzugsweise führen die erfindungsgemäßen Zusammensetzungen und Präparate zu einem vollständigen Ansprechen beim Patienten. Mit vollständigem Ansprechen ist die Beseitigung sämtlicher klinisch nachweisbaren Erkrankungssymptomen sowie die Wiederherstellung von normalen Befunden im Blutbild, bei Röntgenuntersuchungen, CT-Aufnahmen und ähnlichem gemeint. Ein solches Ansprechen dauert mindestens einen Monat nach Absetzen der erfindungsgemäßen Behandlung an. Die antiproliferativ wirksamen Zusammensetzungen und Präparate der vorliegenden Erfindung können zu einem partiellen Ansprechen beim Patienten führen. Bei einem partiellen Ansprechen kommt es zu einer Verringerung der meßbaren Tumorbelastung im Patienten. Dies bedeutet insbesondere, dass die Anzahl der im Patienten vorhandenen Tumorzellen abnimmt und keine neuen Läsionen zu beobachten sind. Gleichzeitig kommt es zu einer Verbesserung eines oder mehrerer durch die Krankheit verursachter Symptome (z.B. Fieber, Gewichtsverlust, Erbrechen).

Die Kombination aus einem oder mehreren Statinen und Astemizol führt zu einer synergistischen, antiproliferativen Wirkung, die einen Einsatz der erfindungsgemäßen Kombination als cytostatisches Mittel bei der Behandlung von Krebs ermöglicht. Wie vorliegend verwendet ist unter einer synergistischen Wirkung eine Wirkung zu verstehen, die stärker ist als durch einfache Addition der bei alleiniger Gabe der Komponenten beobachteten Effekte zu erwarten gewesen wäre. Eine synergistische Wirkung erlaubt die Verabreichung von geringeren Mengen Statin bzw. Astemizol, so dass die Verträglichkeit des erfindungsgemäßen Kombinationspräparat für den Patienten in der Regel problemlos ist. So können die einzelnen Komponenten der Kombination, d.h. das Statin (oder die Statine) und Astemizol, sogar in subtherapeutischen Dosen verabreicht werden, die bei alleiniger Gabe keine Wirkung auf den Verlauf der zu behandelnden Krebserkrankung zeigen würden.

In einem weiteren Aspekt betrifft die Erfindung die Verwendung eines wie oben definierten Statins oder eines pharmazeutisch geeigneten Salzes oder Solvats desselben zur Herstellung einer pharmazeutischen Zusammensetzung, die mindestens ein Statin oder ein pharmazeutisch geeignetes Salz oder Solvat desselben und Astemizol oder ein pharmazeutisch geeignetes Salz oder Solvat desselben umfaßt, für die Behandlung einer Krebserkrankung. Die Erfindung betrifft somit auch die Verwendung von Astemizol oder eines pharmazeutisch geeigneten Salzes oder Solvats desselben zur Herstellung einer pharmazeutischen Zusammensetzung, die mindestens ein Statin oder ein pharmazeutisch geeignetes Salz oder Solvat desselben und Astemizol oder ein pharmazeutisch geeignetes Salz oder Solvat desselben umfaßt, für die Behandlung einer wie oben definierten Krebserkrankung. Entsprechend betrifft die Erfindung auch die Verwendung eines wie oben definierten Statins oder eines pharmazeutisch geeigneten Salzes oder Solvats desselben zur Herstellung eines pharmazeutischen Kombinationspräparats für die gleichzeitige oder zeitlich getrennte Anwendung in der Therapie, wobei das Präparat mindestens ein Statin oder ein pharmazeutisch geeignetes Salz oder Solvat desselben und Astemizol oder ein pharmazeutisch geeignetes Salz oder Solvat desselben umfaßt. Die Erfindung auch die Verwendung von Astemizol oder eines pharmazeutisch geeigneten Salzes oder Solvats desselben zur Herstellung eines pharmazeutischen Kombinationspräparats für die gleichzeitige oder zeitlich getrennte Anwendung in der Therapie, wobei das Präparat mindestens ein Statin oder ein pharmazeutisch geeignetes Salz oder Solvat desselben und Astemizol oder ein pharmazeutisch geeignetes Salz oder Solvat desselben umfaßt.

Bei dem zu verwendenden Statin handelt es sich wie oben beschrieben vorzugsweise um ein Statin aus der Gruppe bestehend aus Atorvastatin, Simavastatin, Cerivastatin, Fluvastatin, Lovastatin, Pravastatin und Rosuvastatin. Besonders bevorzugt wird die Verwendung der Statine Atorvastatin und Lovastatin. Bei der Krebserkrankung, die mit Hilfe des pharmazeutischen Kombinationspräparats behandelt werden soll, kann es sich um eine beliebige der oben aufgeführten Krebserkrankung handeln. Besonders bevorzugt ist es, dass es sich bei der zu behandelnden Krebserkrankung um ein Bronchialkarzinom handelt, insbesondere um ein kleinzelliges Bronchialkarzinom.

Die Erfindung betrifft in einem weiteren Aspekt ein Verfahren zur Behandlung eines Patienten, der an einer Krebserkrankung leidet, bei dem man dem Patienten mindestens ein Statin oder ein pharmazeutisch geeignetes Salz oder Solvat desselben und Astemizol oder ein pharmazeutisch geeignetes Salz oder Solvat desselben verabreicht. Wie oben beschrieben handelt es sich bei dem Statin vorzugsweise um Atorvastatin, Simavastatin, Cerivastatin, Fluvastatin, Lovastatin, Pravastatin oder Rosuvastatin, wobei Atorvastatin oder Lovastatin besonders bevorzugt sind. Die Verabreichung des Statins kann gleichzeitig oder zeitlich getrennt von der Verabreichung von Astemizol erfolgen. Dies bedeutet, dass die Kombination aus Statin und Astemizol wie oben beschrieben entweder in einer einheitlichen Darreichungsform (d.h. in einer einzigen pharmazeutischen Zusammensetzung) vorliegen kann, die an den Patienten verabreicht wird, oder in getrennten Darreichungsformen, die zu verschiedenen Zeitpunkten, d.h. zeitlich getrennt voneinander, an den Patienten verabreicht werden.

In einer bevorzugten Ausführungsform der vorliegenden Erfindung wird das Statin (oder die Statine) vor Verabreichung des Astemizols an den Patienten verabreicht. Vorzugsweise wird die Verabreichung des Statins 1-24 h vor Verabreichung des Astemizols durchgeführt. In einer besonders bevorzugten Ausführungsform wird die Verabreichung des Statins 12-16 h vor Verabreichung des Astemizols durchgeführt. In einer alternativen Ausführungsform der vorliegenden Erfindung wird Astemizol vor Verabreichung des Statins (der Statine) an den Patienten verabreicht. Vorzugsweise wird die Verabreichung von Astemizol 1-24 h vor Verabreichung des Statins durchgeführt. In einer besonders bevorzugten Ausführungsform wird die Verabreichung von Astemizol 12-16 h vor Verabreichung des Statins durchgeführt.

Schließlich betrifft die Erfindung ein Kit, das die folgenden Bestandteile umfaßt:
a) mindestens ein Statin oder ein pharmazeutisch geeignetes Derivat oder Salz desselben,
b) Astemizol oder ein pharmazeutisch geeignetes Derivat oder Salz desselben, und (optional)
c) Anweisungen zur kombinierten Verwendung des Statins und des Astemizols bei der Behandlung eines Patienten mit einer Krebserkrankung.

Die Anweisungen zur kombinierten Verwendung des Statins und Astemizol bei der Behandlung eines Patienten mit einer Krebserkrankung beschreiben dem Anwender die Schritte, die bei der Herstellung und Verabreichung der erfindungsgemäßen pharmazeutischen Zusammensetzungen und Präparate durchzuführen sind, um zu dem gewünschten therapeutischen Ergebnis zu gelangen. Die Anweisungen könne beispielsweise spezifische Mengen und Behandlungspläne umfassen, und sie können Informationen bezüglich der Verabreichungsdauer einschließen. Der Kit kann darüber hinaus andere Bestandteile umfassen, die zur Durchführung der vorliegenden Erfindung nützlich sind. Der Kit kann beispielsweise geeignete Lösungsmittel Hilfsstoffe, Bindemittel, Verdünnungsmittel oder ähnliche Substanzen umfassen. Ferner kann der Kit auch Injektionsspritzen, Kanülen, Katheter und andere Hilfsmittel für die Verabreichung der erfindungsgemäßen pharmazeutischen Zusammensetzungen und Präparate nützlich sind.

Die pharmakologisch wirksamen Substanzen können im Kit in jeder beliebigen Form bereitgestellt werden, die ihre pharmakologische Aktivität erhält. So lassen sich das Statin und das Astemizol beispielsweise in gelöster oder lyophilisierter Form bereitstellen.

### BESCHREIBUNG DER FIGUREN

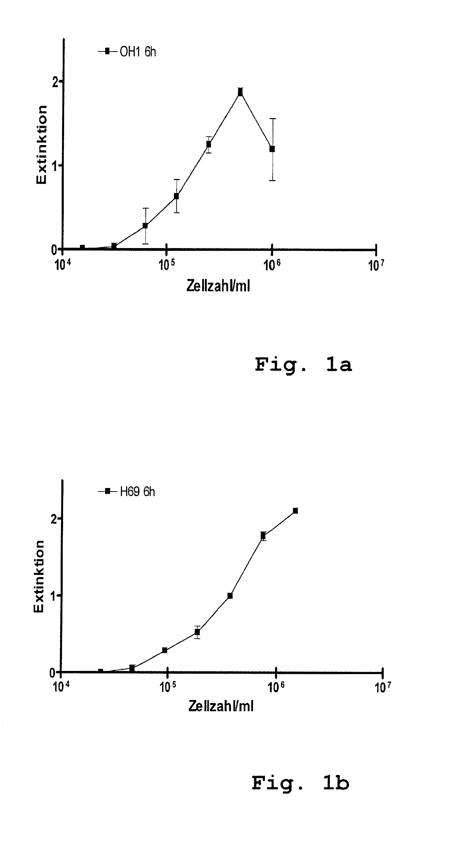

Figur 1a zeigt die Eichkurve für die Zelllinie OH1. Figur 1b zeigt die Eichkurve für die Zelllinie H69.

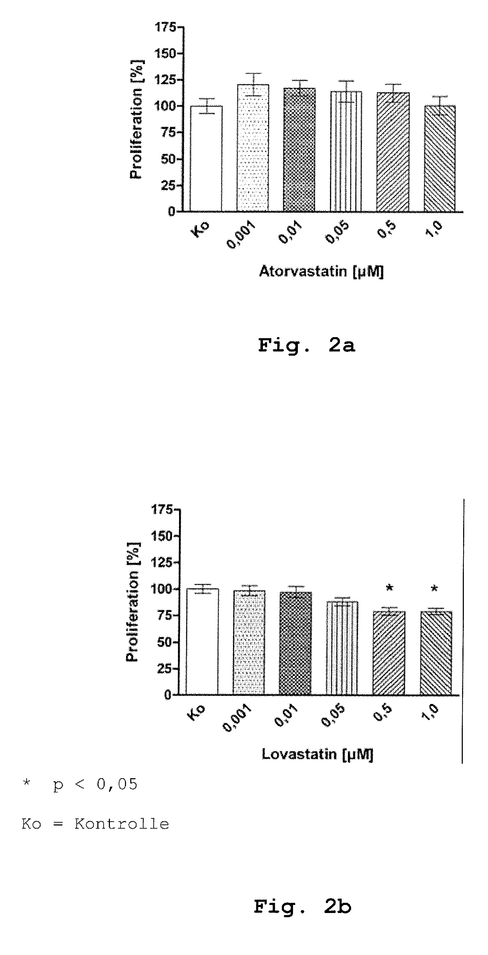

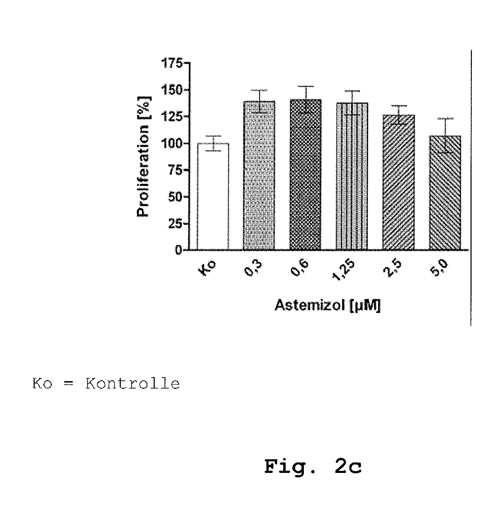

Figur 2 zeigt die Ergebnisse der Wachstumsversuche mit der Zelllinie OH1. Figur 2a zeigt die Ergebnisse der Wachstumsversuche nach Inkubation der Zellen mit verschiedenen Konzentrationen Atorvastatin. Figur 2b zeigt die Ergebnisse der Wachstumsversuche nach Inkubation der Zellen mit verschiedenen Konzentrationen Lovastatin und Figur 2c zeigt die Ergebnisse der Wachstumsversuche nach Inkubation der Zellen mit verschiedenen Konzentrationen Astemizol.

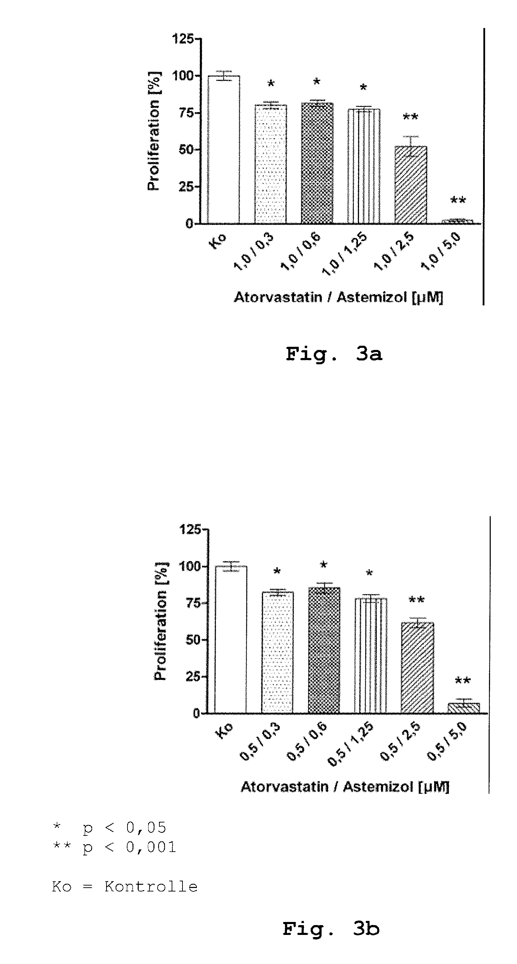

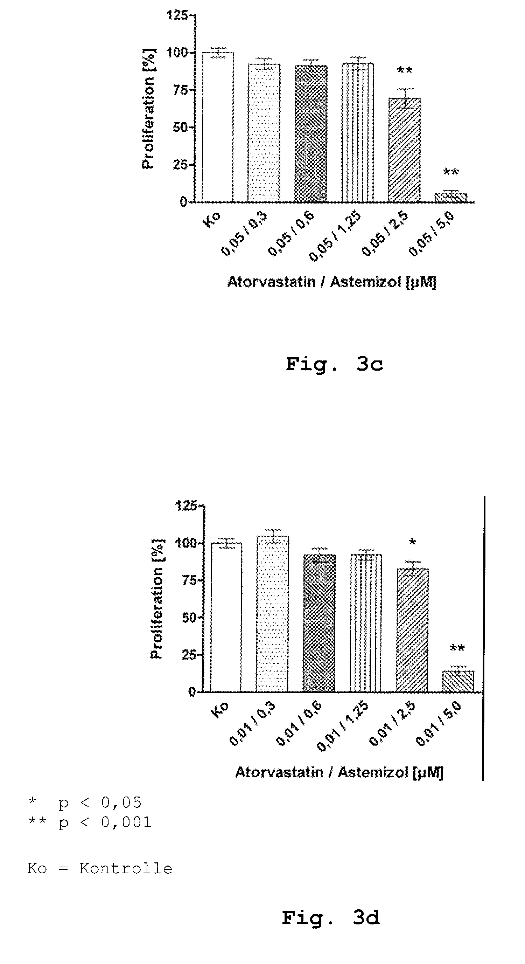

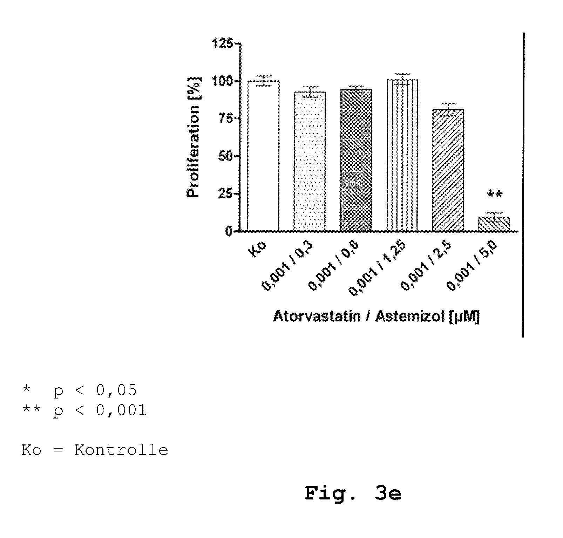

Figur 3 zeigt die Ergebnisse von Wachstumsversuchen mit OH1 Zellen unter Verwendung von unterschiedlichen Konzentration von Atorvastatin und Astemizol. Figur 3a zeigt die Ergebnisse von Wachstumsversuchen in Gegenwart von 1 µM Atorvastatin und variierenden Konzentrationen von Astemizol (0,3 µM bis 5 µM). Figur 3b zeigt die Ergebnisse von Wachstumsversuchen in Gegenwart von 0,5 µM Atorvastatin und variierenden Konzentrationen von Astemizol (0,3 µM bis 5 µM). Figur 3c zeigt die entsprechenden Ergebnisse mit 0,05 µM Atorvastatin, Figur 3d mit 0,01 µM Atorvastatin und Figur 3e mit 0,001 µM Atorvastatin.

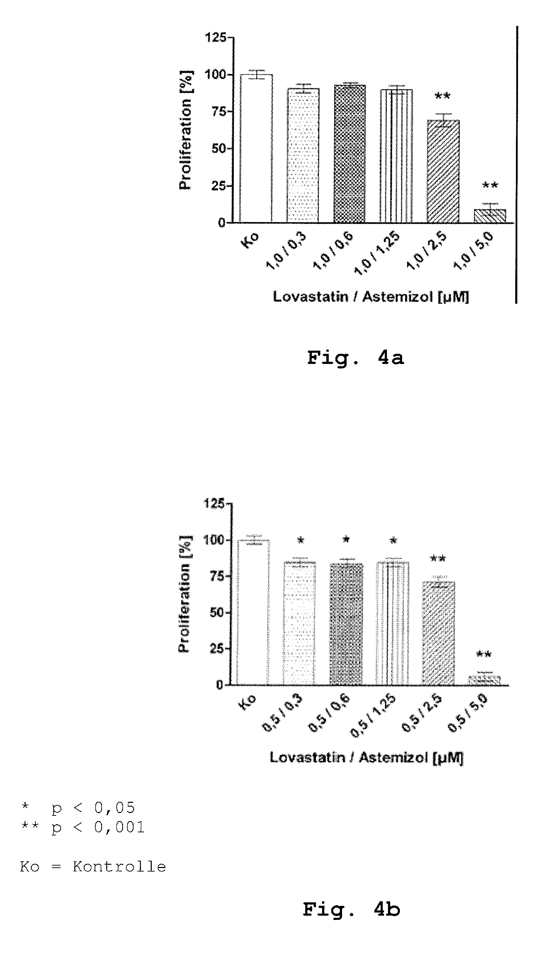

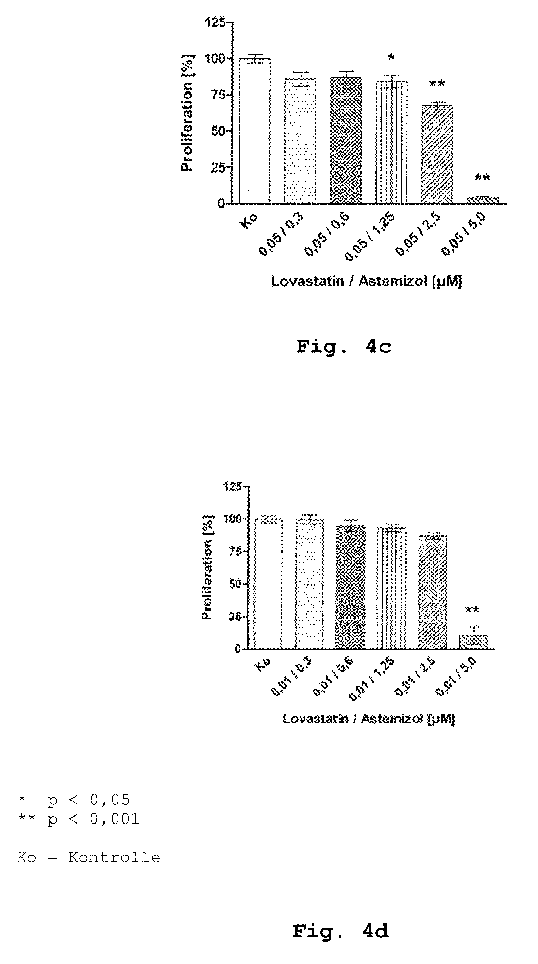

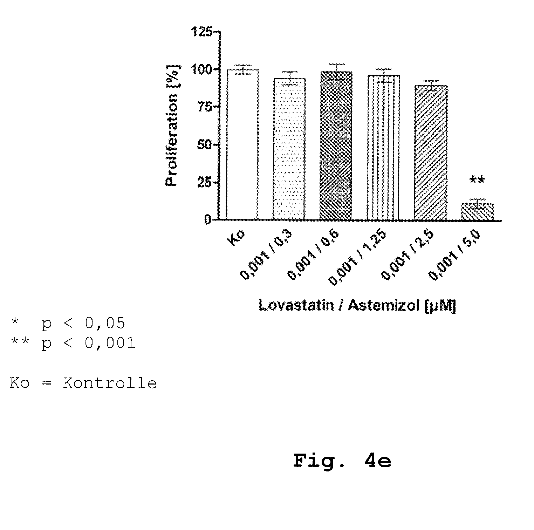

Figur 4 zeigt die Ergebnisse von Wachstumsversuchen mit OH1 Zellen unter Verwendung von unterschiedlichen Konzentrationen von Lovastatin und Astemizol. Figur 4a zeigt die Ergebnisse von Wachstumsversuchen in Gegenwart von 1 µM Lovastatin und variierenden Konzentrationen von Astemizol (0,3 µM bis 5 µM). Figur 4b zeigt die Ergebnisse von Wachstumsversuchen in Gegenwart von 0,5 µM Lovastatin und variierenden Konzentrationen von Astemizol (0,3 µM bis 5 µM). Figur 4c zeigt die entsprechenden Ergebnisse mit 0,05 µM Lovastatin, Figur 4d mit 0,01 µM Lovastatin und Figur 4e mit 0,001 µM Lovastatin.

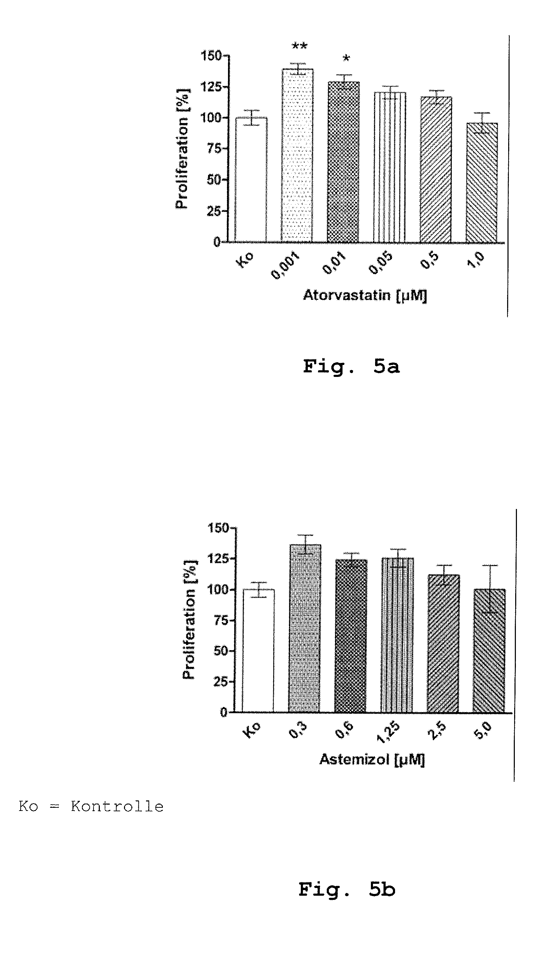

Figur 5 zeigt die Ergebnisse der Wachstumsversuche mit der Zelllinie H69. Figur 5a zeigt die Ergebnisse der Wachstumsversuche nach Inkubation der Zellen mit verschiedenen Konzentrationen Atorvastatin. Figur 5b zeigt die Ergebnisse der Wachstumsversuche nach Inkubation der Zellen mit verschiedenen Konzentrationen Astemizol.

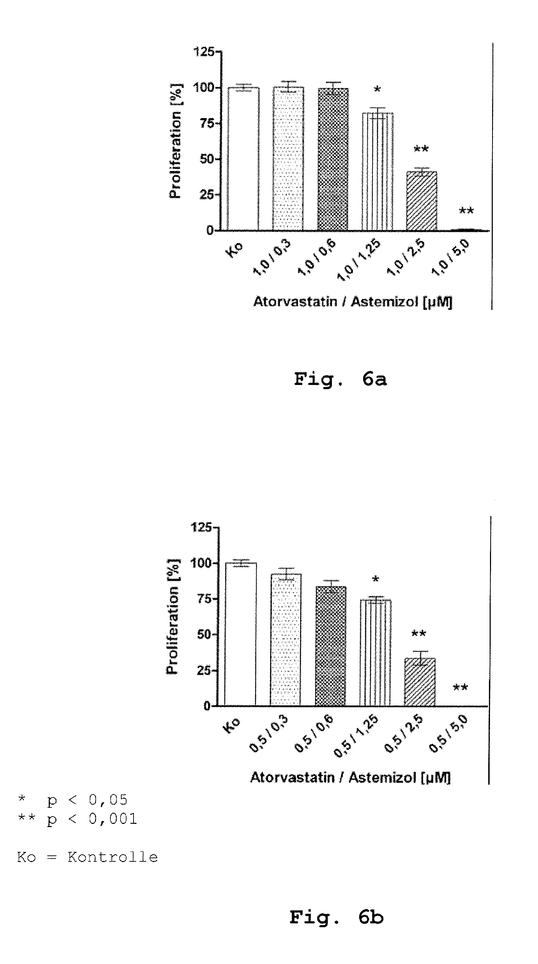

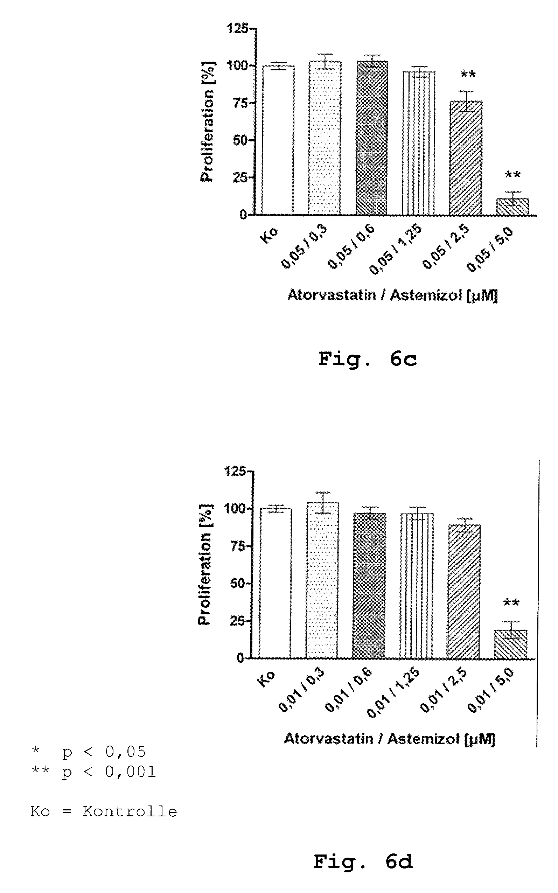

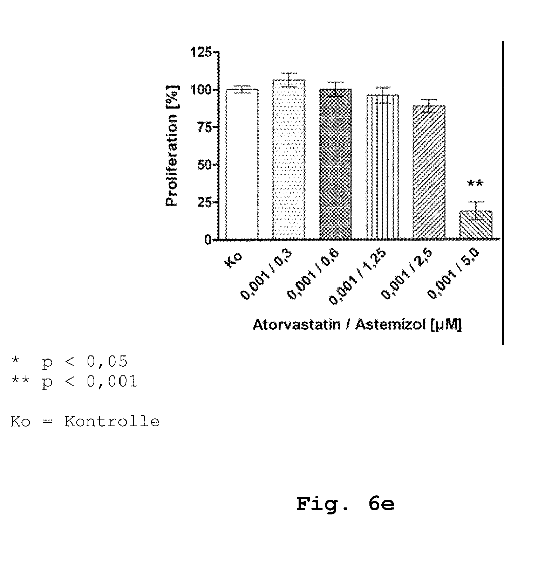

Figur 6 zeigt die Ergebnisse von Wachstumsversuchen mit H69 Zellen unter Verwendung von unterschiedlichen Konzentrationen von Atorvastatin und Astemizol. Figur 6a zeigt die Ergebnisse von Wachstumsversuchen in Gegenwart von 1 µM Atorvastatin und variierenden Konzentrationen von Astemizol (0,3 µM bis 5 µM). Figur 6b zeigt die Ergebnisse von Wachstumsversuchen in Gegenwart von 0,5 µM Atorvastatin und variierenden Konzentrationen von Astemizol (0,3 µM bis 5 µM). Figur 6c zeigt die entsprechenden Ergebnisse mit 0,05 µM Atorvastatin, Figur 6d mit 0,01 µM Atorvastatin und Figur 6e mit 0,001 µM Atorvastatin.

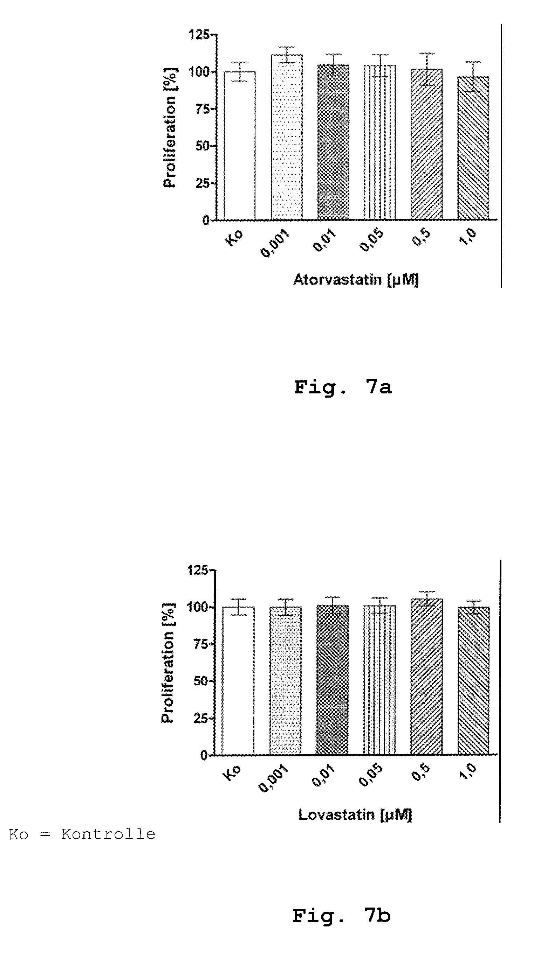

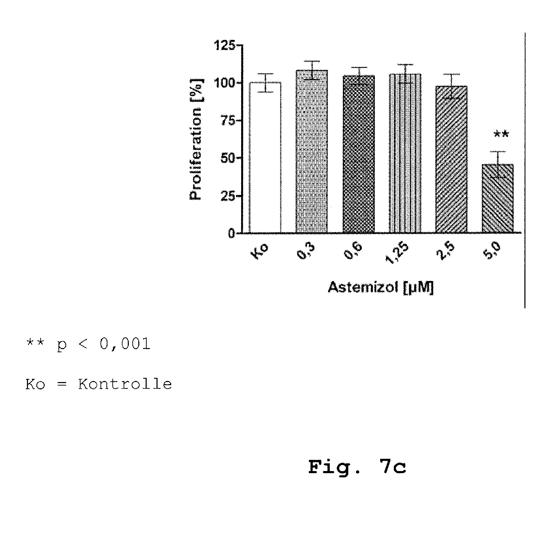

Figur 7 zeigt die Ergebnisse der Wachstumsversuche mit der Zelllinie H82. Figur 7a zeigt die Ergebnisse der Wachstumsversuche nach Inkubation der Zellen mit verschiedenen Konzentrationen Atorvastatin. Figur 7b zeigt die Ergebnisse der Wachstumsversuche nach Inkubation der Zellen mit verschiedenen Konzentrationen Lovastatin und Figur 7c zeigt die Ergebnisse der Wachstumsversuche nach Inkubation der Zellen mit verschiedenen Konzentrationen Astemizol.

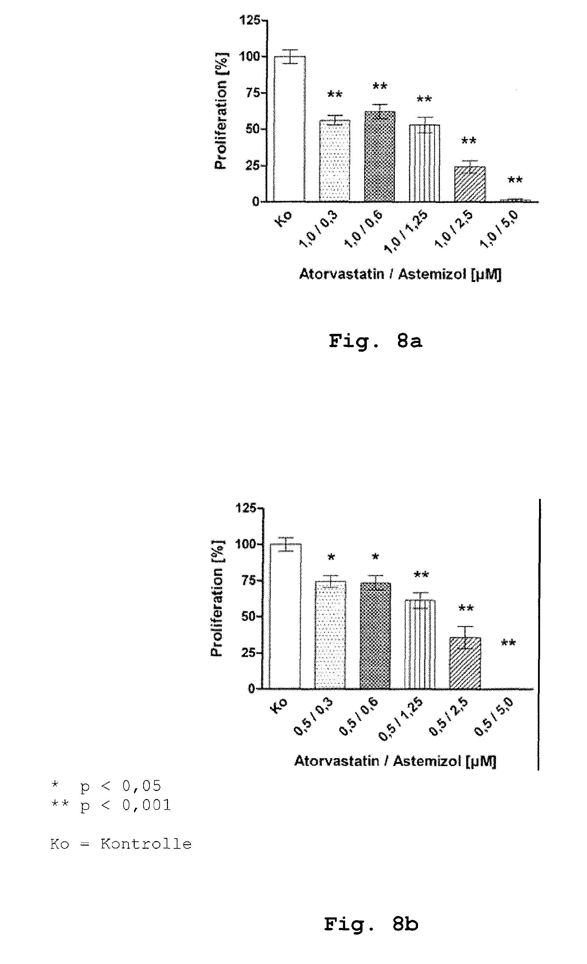

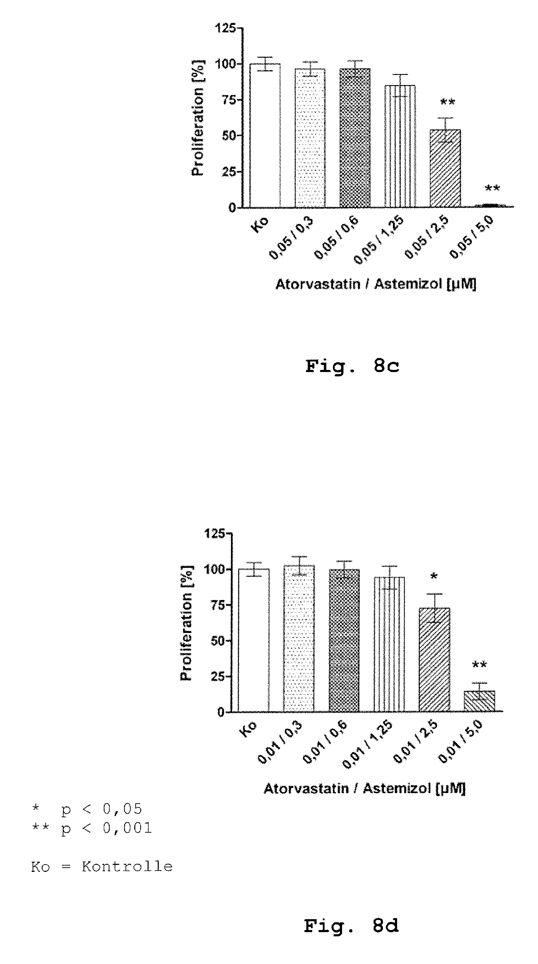

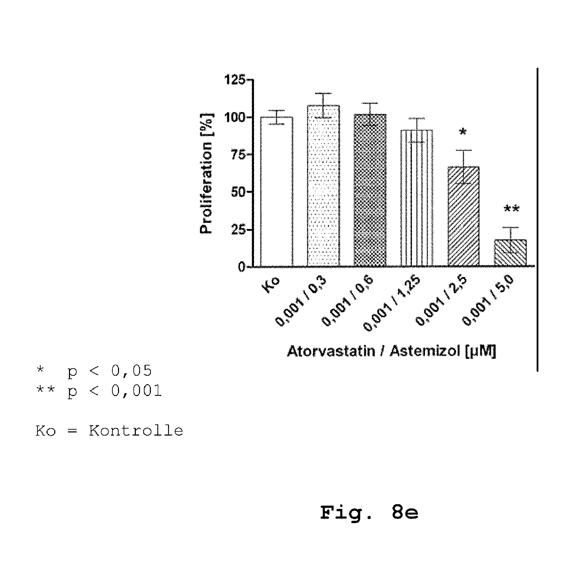

Figur 8 zeigt die Ergebnisse von Wachstumsversuchen mit H82 Zellen unter Verwendung von unterschiedlichen Konzentrationen von Atorvastatin und Astemizol. Figur 8a zeigt die Ergebnisse von Wachstumsversuchen in Gegenwart von 1 µM Atorvastatin und variierenden Konzentrationen von Astemizol (0,3 µM bis 5 µM). Figur 8b zeigt die Ergebnisse von Wachstumsversuchen in Gegenwart von 0,5 µM Atorvastatin und variierenden Konzentrationen von Astemizol (0,3 µM bis 5 µM). Figur 8c zeigt die entsprechenden Ergebnisse mit 0,05 µM Atorvastatin, Figur 8d mit 0,01 µM Atorvastatin und Figur 8e mit 0,001 µM Atorvastatin.

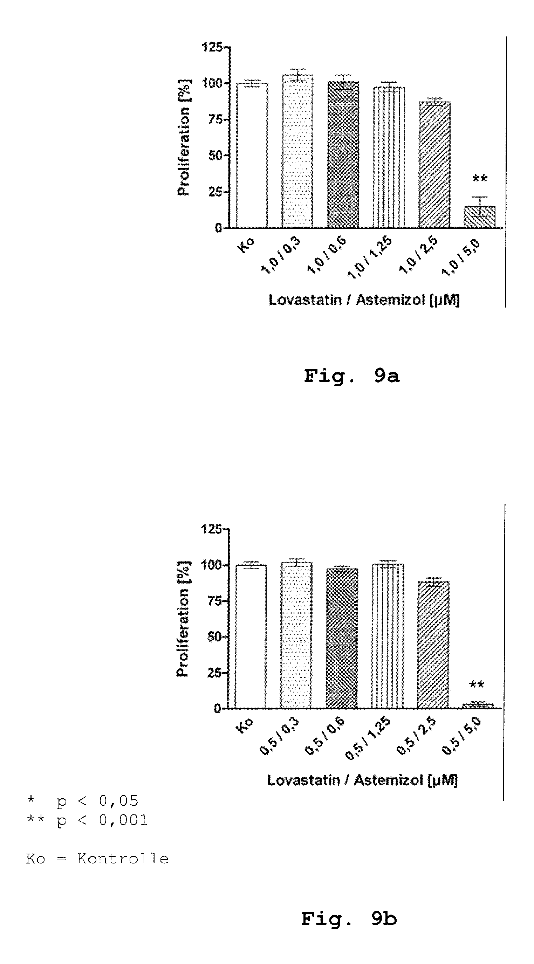

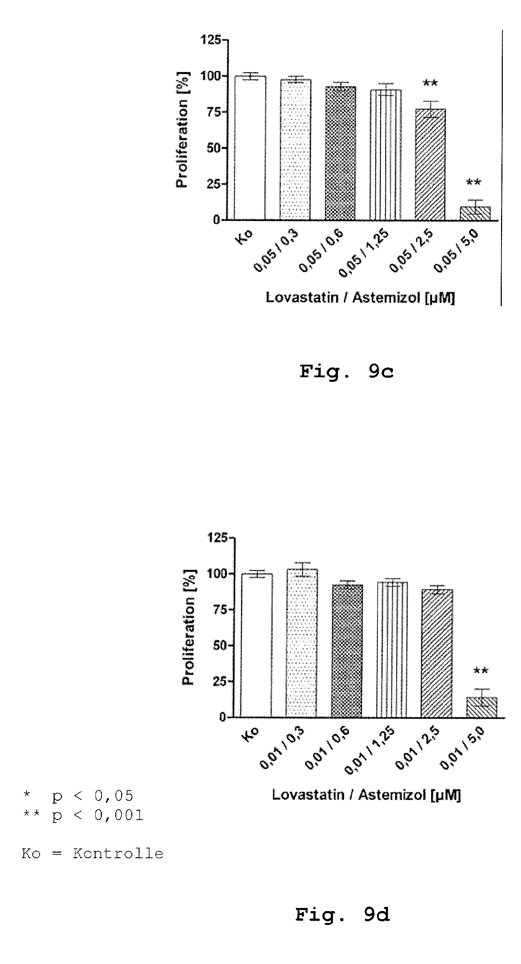

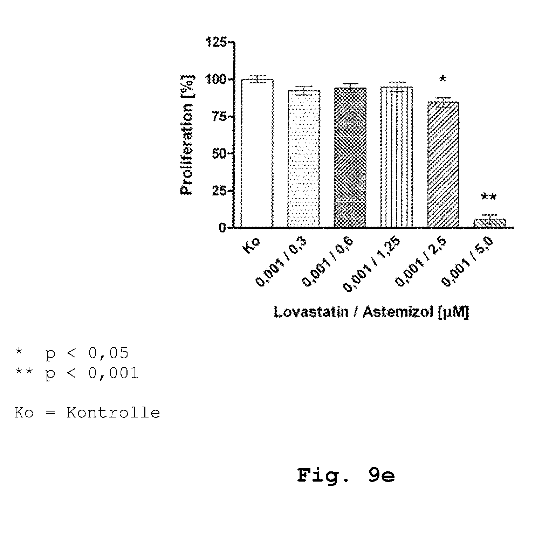

Figur 9 zeigt die Ergebnisse von Wachstumsversuchen mit H82 Zellen unter Verwendung von unterschiedlichen Konzentrationen von Lovastatin und Astemizol. Figur 9a zeigt die Ergebnisse von Wachstumsversuchen in Gegenwart von 1 µM Lovastatin und variierenden Konzentrationen von Astemizol (0,3 µM bis 5 µM). Figur 9b zeigt die Ergebnisse von Wachstumsversuchen in Gegenwart von 0,5 µM Lovastatin und variierenden Konzentrationen von Astemizol (0,3 3 µM bis 5 µM). Figur 9c zeigt die entsprechenden Ergebnisse mit 0,05 µM Lovastatin, Figur 9d mit 0,01 µM Lovastatin und Figur 9e mit 0,001 µM Lovastatin.

Die vorliegende Erfindung wird nunmehr anhand von bevorzugten Ausführungsformen beschrieben, welche die Erfindung veranschaulichen aber in keiner Weise beschränken sollen.

### BEISPIELE

### Beispiel 1: Zellkulturverfahren

Die Wirkung der erfindungsgemäßen pharmazeutischen Zusammensetzungen auf das Wachstum von Tumorzellen wurde an den Zelllinien des kleinzelligen Bronchialkarzinoms OH1, H69 und H82 in Proliferationsversuchen untersucht.

Die Zellen wurden unter *in vitro* Standardbedingungen (37° C, 100% relative Luftfeuchtigkeit, 5% CO₂) in RPMI 1640 mit L-Glutaminzusatz (Gibco) angezüchtet. Einmal wöchentlich wurde das Kulturmedium gegen neues Kulturmedium ausgetauscht. Zu diesem Zweck wurde zunächst der Boden der Zellkulturflasche gründlich abgespült, so dass möglichst alle Zellen im Kulturmedium suspendiert waren. Anschließend wurden aus der Zellkulturflasche ca. 10 ml Zellsuspension abpipettiert und in ein 30 ml Röhrchen überführt. Dieses wurde anschließend für 10 Minuten bei 1500 UpM zentrifugiert. Anschließend wurde der Überstand abgesaugt, und das Zellpellet wurde in frischem Kulturmedium resuspendiert. Die resultierende Zellsuspension wurde in eine neue Zellkulturflasche gegeben, in die zuvor bereits etwa 15 ml Kulturmedium vorgelegt worden waren. Je nach Wachstum der Zellen wurde an zwei weiteren Tagen der Woche eine gewisse Menge der Zellsuspension aus der Zellkulturflasche abgesaugt und frisches Kulturmedium in die Flasche nachgefüllt.

### Beispiel 2: Bestimmung der Zellzahl

Die Anzahl der vitalen Zellen pro 1 ml wurde mit Hilfe eines Zellzählgeräts (Casy®, Cell Counter und Analyzer System Modell TT, Schärfe System, Reutlingen, Deutschland) bestimmt. Dabei werden nach dem Prinzip der Pulsflächen-Analyse Veränderungen in der Membranintegrität detektiert, wodurch tote von vitalen Zellen voneinander unterschieden werden können.

Für die Bestimmung der Zellzahl wurden jeweils etwa 10 ml Zellsuspension in ein 30 ml Röhrchen pipettiert. Dieses wurde bei 1500 UpM für 10 Minuten zentrifugiert. Der Überstand wurde abgesaugt, und das Zellpellet wurde in frischem Kulturmedium resuspendiert. Diese Zellsuspension wurde durch ein Feinsieb ("cell-strainer cap") gegeben, um große aggregierte Zellen voneinander zu trennen. 100 µl der gesiebten Lösung wurden in ein mit 10 ml Zellkultur-Verdünnungslösung Casyton® (Schärfe System, Reutlingen, Deutschland) gefülltes kleines Plastikreagenzgefäß ("Casycup") pipettiert. Nach zweimaligem Invertieren des Casycups wurde mittels des Zellzählgeräts die Zahl der vitalen Zellen bestimmt.

### Beispiel 3: Erstellen von Eichkurven

Vor Beginn der Versuche zur Zellproliferation wurde zunächst für jede Zelllinie eine Eichkurve erstellt, um die optimale Zellzahl pro ml für die nachfolgenden Versuchsreihen zu ermitteln. Dafür wurde das Zellproliferationskit XTT (Roche, Penzberg, Deutschland) verwendet. Hierbei wird durch ein in allen vitalen Zellen vorhandenes Enzym das gelbe Tetrazoliumsalz XTT zu dem orangen Salz Formazan reduziert. Somit stellt die Extinktion der jeweiligen Zellkulturprobe ein Maß für die Anzahl der vitalen Zellen dar.

Die Zelllinien wurden in sieben verschiedenen Konzentrationen zwischen 10⁴ und 10⁷ Zellen pro ml in Mikrotiterplatten mit 96 Vertiefungen ausgesät. In jede Vertiefung wurde zunächst 10 µl PBS (pH 7,4) vorgelegt und dann 90 µl der Zellsuspension mit der jeweiligen Zellzahl pro ml zugegeben. Für jede Zelllinie wurden sechs parallele Ansätze hergestellt. Nach 72 Stunden Inkubation unter Standardbedingungen wurden 50 µl XTT Labeling Mixture pro Vertiefung zugegeben. Nach sechs Stunden wurde die Absorption bei 450 nm in einem Dynatech MR 3.13 Mikroelisa Reader gemessen und anhand der erzielten Werte Eichkurven erstellt. Figur 1a zeigt die Eichkurve für OH1-Zellen; Figur 1b zeigt die Eichkurve für H69-Zellen. Die Zellzahlen wurden so gewählt, dass sich die Zellen bei ungehindertem Wachstum in der exponentiellen Wachstumsphase befanden. So ergab sich eine Konzentration von 300.000 Zellen pro ml für OH1 bzw. 500.000 Zellen pro ml für H69.

### Beispiel 4: Wirkungstests mit OH1-Zellen

Es wurden die Auswirkungen von Atorvastatin, Lovastatin und Astemizol (allein oder in Kombination) auf das Wachstum von OH1-Zellen analysiert. Für die Versuche wurde das oben beschriebene Kulturmedium mit verschiedenen Konzentrationen (1 µM, 0,5 µM, 0,05 µM, 0,01 µM und 0,001 µM) Atorvastatin, Lovastatin bzw. Astemizol ergänzt und anschließend steril filtriert. Als Kontrolle wurde Kulturmedium ohne Zusatz von Statin oder Astemizol verwendet.

Das Atorvastatin wurde aus Tabletten gewonnen, wie es in Mühlhäuser et al. (Mühlhäuser et al., FASEB Journal (2006), 20 (6), 785-787 beschrieben ist.

Die Zellen wurden in einer Menge von 150.000 Zellen pro ml in Mikrotiterplatten mit 96 Vertiefungen (100 µl pro Vertiefung) ausgesät. Um das Zellwachstum in den Medien zu vergleichen, wurden pro Platte jeweils vier Ansätze der OH1 Zelllinie in Kulturmedium ausgesät und vier Ansätze in dem Medium, das mit Atorvastatin, mit Lovastatin oder mit Astemizol ergänzt worden war. Nach einer Inkubation von 72 Stunden wurden 50 µl XTT-Lösung pro Vertiefung hinzugegeben. Nach weiteren sechs Stunden wurde die Absorption bei 450 nm gemessen. Die Versuche wurden insgesamt dreimal durchgeführt, so dass sich jeder Messpunkt aus 12 einzelnen Meßwerten zusammensetzte.

Die Ergebnisse der Versuche sind in Figur 2 dargestellt. Figur 2a zeigt die Ergebnisse für die Versuche, bei denen die OH1-Zellen mit Atorvastatin versetzt wurden. In Figur 2b sind die Ergebnisse für die Versuche gezeigt, in denen die OH1-Zellen mit Lovastatin behandelt wurden und in Figur 2c sind die Ergebnisse für die Versuche gezeigt, in denen die OH1-Zellen mit Astemizol behandelt wurden. Man erkennt deutlich, dass die alleinige Gabe von Atorvastatin, Lovastatin bzw. Astemizol nicht zu einer Verringerung des Wachstums der OH1-Zellen führt.

In weiteren Versuchsreihen wurde die Wirkung der erfindungsgemäßen Kombinationen aus dem Statin Atorvastatin bzw. Lovastatin und Astemizol auf das Wachstum der OH-1 Zellen untersucht. Hierzu wurden die Konzentrationen von Atorvastatin bzw. Lovastatin und Astemizol sukzessive erhöht, um die Auswirkungen der veränderten Konzentrationen auf das Zellwachstum zu verfolgen. In einer ersten Versuchsreihe wurde jeweils 1 µM Atorvastatin mit 5 µM, 2,5 µM, 1,25 µM, 0,6 µM oder 0,3 µM Astemizol wie oben beschrieben eingesetzt. Die Ergebnisse sind in Figur 3a gezeigt. In weiteren Versuchsreihen wurde die Konzentration von Atorvastatin immer weiter verringert. Figur 3b zeigt die Ergebnisse der Wachstumsversuche mit 0,5 µM Atorvastatin. Figur 3c zeigt die Ergebnisse der Wachstumsversuche mit 0,05 µM Atorvastatin, Figur 3d die Ergebnisse mit 0,01 µM Atorvastatin, und Figur 3e die Ergebnisse mit 0,001 µM Atorvastatin. Die Konzentrationen von Astemizol entsprachen jeweils den oben aufgeführten (5 µM, 2,5 µM, 1,25 µM, 0,6 µM oder 0,3 µM). Die Ergebnisse der entsprechenden Versuche mit einer Kombination von Lovastatin und Astemizol sind in den Figuren 4a- 4e gezeigt.

Die Ergebnisse der Versuche zeigen zunächst, dass es durch die kombinierte Gabe von Atorvastatin bzw. Lovastatin mit Astemizol zu einer deutlichen Verringerung des Zellwachstums der Krebszellen im Vergleich zur alleinigen Gabe der jeweiligen Substanzen kommt. Die stärkste Hemmung des Zellwachstums wurde erreicht, wenn eine Konzentration von 5 µM Astemizol gewählt wurde.

### Beispiel 5: Wirkungstests mit H69-Zellen

Die in Beispiel 4 beschriebenen Versuchsreihen wurden mit der Zelllinie H69 wiederholt, wobei hier nur Atorvastatin und Astemizol eingesetzt wurden. Die Ergebnisse sind in den Figuren 5 und 6 gezeigt.

Auch in diesem Fall zeigen die in den Figuren 5a und 5b dargestellten Ergebnisse, dass die alleinige Gabe von Atorvastatin oder Astemizol nicht zu einer Verringerung des Wachstums der Zellen führt. Die in den Figuren 6a-6e dargestellten Ergebnisse zeigen dagegen die Wirksamkeit der kombinierten Gabe von Atorvastatin mit Astemizol bei der Hemmung des Wachstums der Krebszellen. Die stärkste Hemmung wurde bei einer Astemizol-Konzentration von 5 µM erreicht. Ferner zeigte sich, dass der antiproliferative Effekt bei kombinierter Gabe von Atorvastatin und Astemizol bei einer Verringerung der Atorvastatin-Konzentration auf unter 0,5 µM schwächer wird.

### Beispiel 6: Wirkungstests mit H82-Zellen

Die in Beispiel 4 beschriebenen Versuchsreihen wurden mit der Zelllinie H82 wiederholt. Die Ergebnisse sind in den Figuren 7 bis 9 gezeigt.

Wie bei den Versuchen zuvor, hatte die alleinige Gabe von Atorvastatin, Lovastatin bzw. Astemizol kaum Auswirkungen auf das Zellwachstum (einzige Ausnahme ist die Gabe von Astemizol mit einer Konzentration von 5 µM, die zu einer 50%igen Hemmung der Proliferation führte). Die kombinierte Gabe von Atorvastatin mit Astemizol (siehe Figuren 8a-8e) bzw. Lovastatin mit Astemizol (s. Figuren 9a-9e) führte zu einer drastischen Verringerung der Proliferation der H82 Zellen.

Die obigen Ergebnisse der in vitro-Versuche zeigen deutlich, dass es bei einer kombinierten Gabe von Atorvastatin und Astemizol bzw. Lovastatin und Astemizol zu einem starken synergistischen Effekt kommt, der eine wesentlich stärkere Hemmung von Krebszellen bewirkt, als durch die Verwendung der einzelnen Substanzen zu erreichen wäre.

## Patentansprüche

1. Pharmazeutische Zusammensetzung, umfassend:
a) mindestens ein Statin oder ein pharmazeutisch geeignetes Salz oder Solvat desselben, und
b) Astemizol oder ein pharmazeutisch geeignetes Salz oder Solvat desselben.

2. Pharmazeutische Zusammensetzung gemäß Anspruch 1, **dadurch gekennzeichnet, dass** das Statin ausgewählt ist aus der Gruppe, bestehend aus Atorvastatin, Simavastatin, Cerivastatin, Fluvastatin, Lovastatin, Pravastatin und Rosuvastatin.

3. Pharmazeutische Zusammensetzung gemäß Anspruch 2, **dadurch gekennzeichnet, dass**, **dadurch gekennzeichnet, dass** das Statin Atorvastatin oder Lovastatin ist.

4. Pharmazeutische Zusammensetzung gemäß einem der Ansprüche 1 bis 3, **dadurch gekennzeichnet, dass** sie ferner einen pharmazeutisch annehmbaren Träger oder Hilfsstoff umfaßt.

5. Pharmazeutische Zusammensetzung gemäß einem der Ansprüche 1 bis 4 für die Behandlung einer Krebserkrankung.

6. Pharmazeutische Zusammensetzung gemäß Anspruch 5, **dadurch gekennzeichnet, dass** die Krebserkrankung ein Bronchialkarzinom ist.

7. Pharmazeutische Zusammensetzung gemäß Anspruch 6, **dadurch gekennzeichnet, dass** die Krebserkrankung ein kleinzelliges Bronchialkarzinom ist.

8. Verwendung eines Statins oder eines pharmazeutisch geeigneten Salzes oder Solvats desselben zur Herstellung einer pharmazeutischen Zusammensetzung für die Behandlung einer Krebserkrankung, wobei die Zusammensetzung mindestens ein Statin oder ein pharmazeutisch geeignetes Salz oder Solvat desselben und Astemizol oder ein pharmazeutisch geeignetes Salz oder Solvat desselben umfaßt.

9. Verwendung von Astemizol oder eines pharmazeutisch geeigneten Salzes oder Solvats desselben zur Herstellung einer pharmazeutischen Zusammensetzung, für die Behandlung einer Krebserkrankung, wobei die Zusammensetzung mindestens ein Statin oder ein pharmazeutisch geeignetes Salz oder Solvat desselben und Astemizol oder ein pharmazeutisch geeignetes Salz oder Solvat desselben umfaßt.

10. Verwendung gemäß einem der Ansprüche 8 oder 9, **dadurch gekennzeichnet, dass** das Statin ausgewählt ist aus der Gruppe bestehend aus Atorvastatin, Simavastatin, Cerivastatin, Fluvastatin, Lovastatin, Pravastatin und Rosuvastatin.

11. Verwendung gemäß Anspruch 10, **dadurch gekennzeichnet, dass** das Statin Atorvastatin oder Lovastatin ist.

12. Pharmazeutisches Kombinationspräparat, umfassend:
a) mindestens ein Statin oder ein pharmazeutisch geeignetes Salz oder Solvat desselben, und
b) Astemizol oder ein pharmazeutisch geeignetes Salz oder Solvat desselben, für die gleichzeitige oder zeitlich getrennte Anwendung in der Therapie.

13. Pharmazeutisches Kombinationspräparat gemäß Anspruch 12, **dadurch gekennzeichnet, dass** das Statin oder das pharmazeutisch geeignete Salz oder Solvat desselben und Astemizol oder das pharmazeutisch geeignete Salz oder Solvat desselben für die getrennte Verabreichung formuliert sind.

14. Pharmazeutisches Kombinationspräparat gemäß Anspruch 13, **dadurch gekennzeichnet, dass** das Statin oder das pharmazeutisch geeignete Salz oder Solvat desselben und Astemizol oder das pharmazeutisch geeignete Salz oder Solvat desselben in unterschiedlichen Darreichungsformen formuliert sind.

15. Kit, umfassend
a) mindestens ein Statin oder ein pharmazeutisch geeignetes Derivat oder Salz desselben,
b) Astemizol oder ein pharmazeutisch geeignetes Derivat oder Salz desselben.
